(19) Europäisches Patentamt
European Patent Office
Office européen des brevets

(11) **EP 2 906 291 B1**

(12) **EUROPEAN PATENT SPECIFICATION**

(45) Date of publication and mention of the grant of the patent:
**15.05.2019 Bulletin 2019/20**

(21) Application number: **13814610.5**

(22) Date of filing: **30.09.2013**

(51) Int Cl.:
*A61N 7/02* *(2006.01)*     *A61B 34/10* *(2016.01)*

(86) International application number:
**PCT/IB2013/059001**

(87) International publication number:
**WO 2014/057388 (17.04.2014 Gazette 2014/16)**

(54) **MULTI-FOCI SONICATIONS FOR HYPERTHERMIA TREATMENTS USING MAGNETIC RESONANCE-GUIDED FOCUSSED ULTRASOUND**

MULTIFOKALE HYPERTHERMIEBEHANDLUNG MIT MAGNETRESONANZGESTEUERTEM FOKUSSIERTEM ULTRASCHALL

ULTRASONIFICATION À FOYERS MULTIPLES POUR TRAITEMENT DE L'HYPERTHERMIE UTILISANT UN ULTRASON FOCALISÉ GUIDÉ PAR RÉSONANCE MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2012 US 201261713132 P**

(43) Date of publication of application:
**19.08.2015 Bulletin 2015/34**

(73) Proprietors:
• **Profound Medical Inc.**
  **Mississauga, ON L4W 5K5 (CA)**
• **The Government of the United States**
  **As represented by the Secretary of Health & Human**
  **Services,**
  **Bethesda, MD 20892 (US)**

(72) Inventors:
• **PARTANEN, Ari Ilkka Mikael**
  **Bethesda, MD 20817 (US)**
• **TILLANDER, Matti Oskari**
  **01630 Vantaa (FI)**

• **DREHER, Matthew**
  **5656 AE Eindhoven (NL)**
• **KÖHLER, Max**
  **5656 AE Eindhoven (NL)**

(74) Representative: **Schnekenbühl, Robert et al**
  **DTS Patent- und Rechtsanwälte**
  **Schnekenbühl und Partner mbB**
  **Marstallstraße 8**
  **80539 München (DE)**

(56) References cited:
**WO-A2-2011/156624     US-A- 4 893 624**
**US-A- 5 524 625       US-A1- 2010 030 076**
**US-A1- 2012 191 020**

• **LALONDE R J ET AL: "OPTIMIZING ULTRASOUND FOCUS DISTRIBUTIONS FOR HYPERTHERMIA", IEEE TRANSACTIONS ON BIOMEDICAL ENGINEERING, IEEE SERVICE CENTER, PISCATAWAY, NJ, USA, vol. 42, no. 10, 1 October 1995 (1995-10-01), pages 981-990, XP000556830, ISSN: 0018-9294, DOI: 10.1109/10.464372**

**Description**

The present application relates to apparatus for hyperthermia treatment using multi-foci sonications.

Mild hyperthermia (HT) is a therapeutic technique in which tissue is heated to temperatures above body temperature and below ablative temperatures, for example, 38-45°C. These mild hyperthermia treatments may result in physiological, e.g., perfusion, and cellular, e.g., gene expression, changes that improve the therapeutic effectiveness when used in conjunction with chemotherapy or radiation therapy. Mild hyperthermia induces a multitude of changes, which provide clinical benefits that make it synergistic with many chemotherapeutic agents and radiation therapy. In addition to physiological and cellular changes, hyperthermia may be used with temperature responsive, as well as non-responsive, drug delivery systems to reduce toxicity and improve overall efficacy. One solution for reducing toxicity involves targeting the tumor with temperature sensitive liposomal drug delivery. At normal body temperature (~37°C) temperature sensitive liposomes are relatively stable, while at mild hyperthermic temperatures of about 38-45°C, temperature sensitive liposomes exhibit drug release within 10-20 seconds.

There are a number of currently available devices that can heat target tissue to the mild hyperthermic range. One example is radiofrequency (RF) applicators, which are tuned antennas to transmit RF energy into the body. RF applicators are best used to heat deep-seated tumors due the long wavelengths of RF. Microwave applicators are also used, but are typically used only for superficial tumors due to their short wavelength. Hot water baths, lasers, and magnetic fluids have also been used. These suffer from drawbacks such as invasive nature, limited or superficial heating, hot and cold spots, inaccurate or spatially uneven heating, and a lack of spatiotemporal feedback control. Local hyperthermia is also performed by magnetic resonance-guided high intensity focused ultrasound (MR-HIFU), in which a focused ultrasound spot is swept rapidly to achieve hyperthermia and MR is used to monitor the treatment. The focused spot tends to temporarily heat tissue to temperatures above the target temperature at the exact focal spot location before moving to the next location. Moreover, single, high intensity focal spots may also more easily cause deleterious mechanical tissue damage.

Using high intensity focused ultrasound can result in both thermal and non-thermal (mechanical) bioeffects, both of which arise from a complex interaction of propagating ultrasound waves with tissue. High intensity focused ultrasound (HIFU) bioeffects can be manipulated and/or controlled by ultrasound output power, frequency, duty cycle, sonication duration, and focal spot characteristics. Thermal effects due to ultrasound absorption and conversion to heat through vibrational excitation of tissue lead to rapid, highly localized temperature elevation. The mechanical effects that are unique to HIFU include acoustic radiation forces and acoustic cavitation. The radiation forces may lead to local tissue displacement, shear strain, and streaming, while cavitation effects are mediated by bubble activity - collapsing or oscillating bubbles lead to locally induced stress and high energy release, possibly resulting in and assisting thermal coagulation and necrosis. The mechanical ultrasound effects may be exploited to improve ablation efficiency, or improve drug delivery with or without the addition of micro bubbles in certain applications.

MRI provides in vivo temperature maps during a HIFU sonication. Real-time evaluation of temperature elevations can be monitored in the temperature maps and the power, duration, frequency or trajectory (i.e. the spatiotemporal pattern) of the sonication can be adjusted accordingly.

The present application presents approaches for sustained mild hyperthermia over an extended period of time using a multi-foci sonication approach to control temperature accuracy and uniformity, to confine heated volumes, and to lower peak acoustic pressures in the heated region. The present application refines the multi-foci heating strategy as a tool for mild hyperthermia applications, such as in clinical oncology. However, the multi-foci heating strategy may also have other applications such as in MR-HIFU thermal ablations.

United States patent application US 2012/0191020 A1 diclsoes systems and methods for heating a surface substantially uniformly are provided. In various embodiments, the uniform heating is achieved by moving an ultrasound beam across the surface and/or by sequentially irradiating individual meshes of a mesh grid defined over the surface.

United States patent application US 2010/0030076 A1 diclsoes the emission intensities of groupings of transducer elements of an ultrasound transducer array are controlled based on targeting criteria in such a manner as to simultaneously create multiple discontiguous foci, each corresponding to one of a plurality of target sites. The invention is defined by the appended independent claim. Preferred embodiments are provided in the dependent claims. In accordance with the invention, a mild hyperthermia treatment apparatus is provided. The apparatus includes an imager, which generates a planning image and temperature maps of a target region. The apparatus further includes a phased array of ultrasonic transducers. An array of ultrasonic transducer drivers individually drives the ultrasonic transducers of the phased array to generate multi-foci sonications in the target region. One or more processors are programmed to receive a target temperature profile and calculate power, frequency, and relative phase for the transducer drivers of the ultrasonic transducer array which is calculated to cause the phased array of ultrasonic transducers to generate a multi-foci sonication pattern configured to heat the target region with the target temperature profile.

In accordance with another aspect, a mild hyperthermia treatment apparatus is provided which includes an imager and a scan controller which controls the imager to generate a planning image and temperature maps of a target region. An array of ultrasonic transducer drivers drives a phased array of ultrasonic transducers individually to generate multiple, simultaneous focal spots in a target region. A planning console displays the planning image and has an input by which a clinician inputs a target temperature profile and a target location (area or volume). An ultrasound controller receives the target temperature profile and calculates power, frequency, and relative phase for the transducer drivers which are calculated to cause the phased array of ultrasonic transducers to generate a multi-foci sonication pattern to heat the target region to the target temperature profile.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.

FIG. 1 is a diagrammatic illustration of a system for magnetic resonance-guided multi-foci sonications for mild hyperthermia treatments;

FIG. 2A is a simulated acoustic pressure field for a single point deflection 4 mm to the left of center;

FIG. 2B is a simulation of a multi-foci sonication with 16 simultaneous foci spaced evenly at a circle with an 8 mm diameter;

FIG. 2C illustrates a sagittal image plane, i.e., in the beam path direction, corresponding to the single point sonication of FIG. 2A;

FIG. 2D is a sagittal image plane image corresponding to the multi-foci sonication pattern of FIG. 2B;

FIG. 3A illustrates an acoustic pressure map for a single focal point sonication 4 mm to the left of the image origin coordinates;

FIG. 3B illustrates an acoustic pressure map for the 16 foci pattern of FIG. 2B;

FIG. 3C illustrates a phase map for the single focus sonication of FIG. 3A;

FIG. 3D illustrates a phase map of the 16 foci pattern of FIG. 3B;

FIG. 4A illustrates a simulated coronal temperature distribution map obtained by using an 8 mm sonication trajectory in which a single point is swept temporally across 16 focal points sufficiently rapidly to achieve effective thermal averaging;

FIG. 4B illustrates the sagittal temperature distribution map from the sonication of FIG. 4A;

FIG. 4C illustrates a simulated coronal temperature distribution map obtained by using a multi-foci sonication pattern with 16 concurrent foci;

FIG. 4D illustrates a sagittal simulated temperature distribution map from the multi-foci sonication pattern of FIG. 4C;

FIG. 5A illustrates coronal tumor mapping on a proton density weighted planning image;

FIG. 5B illustrates a coronal temperature map of achieved temperatures overlaid on the planning image during mild hyperthermia treatment showing a typical temperature distribution after 3 minutes of heating;

FIG. 5C is a sagittal image corresponding to FIG. 5A;

FIG. 5D is a sagittal temperature map corresponding to the coronal temperature distribution map of FIG. 5B;

FIG. 6A illustrates mean (solid), 10th percentile, and 90th percentile (dashed) temperatures within an 8 mm treatment cell over a 5 minute sonication *in vivo* using a single focus sweep approach;

FIG. 6B illustrates mean (solid), and 10th and 90th percentile (dashed) temperatures within an 8 mm treatment cell over a 5 minute sonication using a multi-foci approach in which the target temperature range is indicated as a gray box;

FIG. 7 illustrates a time-average mean temperature radial line profile centered on an 8 mm treatment cell of a tumor for both single focus (solid) and multi-foci (dashed) sonication approaches;

FIG. 8A illustrates a time-averaged spatial temperature distribution in a coronal plane for an 8 mm treatment cell for a single focus sweep sonication approach within a tumor;

FIG. 8B illustrates a time-averaged spatial temperature distribution in a coronal plane for an 8 mm treatment cell using a multi-foci sonication approach within a tumor;

FIG. 8C illustrates the time-averaged temperature distribution in the sagittal plane corresponding to FIG. 8A;

FIG. 8D illustrates the time-averaged spatial temperature distribution in the sagittal plane corresponding to FIG. 8B; and

FIG. 9 is a flow chart illustrating computer processing or treatment steps for multi-foci sonication induced mild hyperthermia.

The optimal temperature for most mild hyperthermia applications is in the 40-45°C. range. Temperatures below 40°C typically have limited effect and temperatures greater than 45°C may shut down tissue perfusion. By contrast, in ablation techniques, the temperature is raised to level capable of inducing necrosis, typically greater than 55°C, and then letting the tissue cool. Overheating of the target tissue to be ablated is not detrimental. The present mild hyperthermia technique maintains the temperature in the target region at the desired level, e.g., between 40.5°C and 41.0°C, for a long duration, typically 3-45 minutes or more. During this time, the power, frequency, and trajectory are adjusted to obtain an optimal heating uniformity in the target volume for e.g. homogeneous tissue sensitization or drug delivery.

The effects of HIFU on tissue are highly dependent on the acoustic intensity of the focal region, with different mechanisms of HIFU energy propagation dominating at high and low acoustic intensities. When operating at low acoustic intensities, the acoustic field is predominantly linear, the waves have harmonic shape, and acoustic intensity is proportional to the pressure squared. At the higher acoustic intensity levels, the peak negative (rarefaction) pressure correlates well with the onset of cavitation effects and nonlinear wave propagation leads to the generation of higher harmonics, asymmetric distortion of pressure waveforms, and ultimately, to the forming of steep shock fronts. The nonlinear broadening of the spectrum to higher frequencies and the formation of shocks can significantly increase local absorption of the acoustic energy, especially in the focal region. This absorption of acoustic energy drastically increases the local heating rate. The unpredictable nature of nonlinear acoustic propagation and mechanical bioeffects raise potential concerns for clinical applications in a diverse patient population, even when a focus point of high intensity is scanned rapidly. Mild hyperthermia attempts to avoid direct tissue damage due to both mechanical and thermal bioeffects. Rather, mild hyperthermia aims at only sensitizing the tissue to adjuvant therapies, such as thermally activated drug therapies.

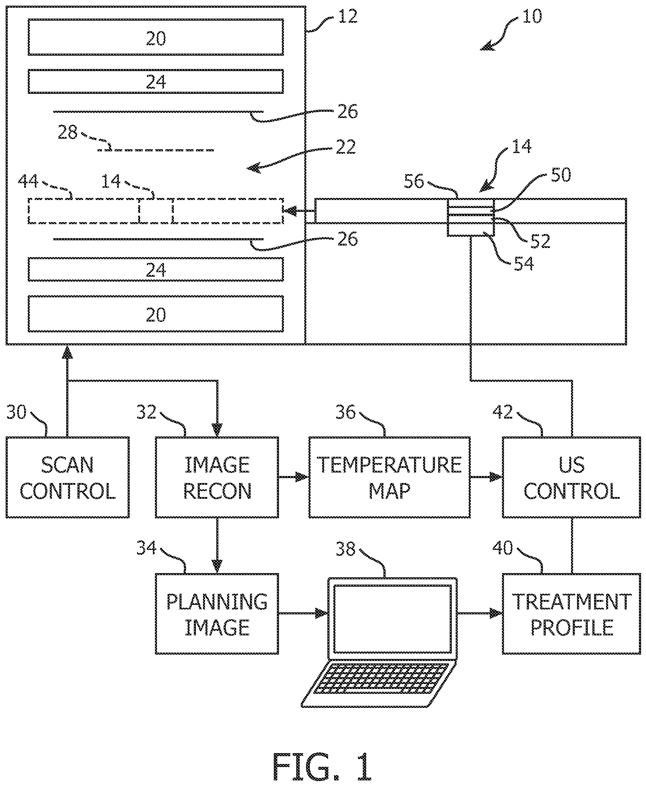

With reference to FIG. 1, a magnetic resonance guided mild hyperthermia treatment system 10 includes a magnetic resonance imaging system 12 and an ultrasonic probe 14. The magnetic resonance imaging system includes a main magnet 20 for generating a temporally constant $B_0$ main magnetic field through an imaging region 22. Gradient magnetic coils 24 are selectively pulsed to generate magnetic field gradients across the main magnetic field. Whole body radio frequency (RF) coils 26 are controlled to induce magnetic resonance in a subject in the imaging region. The induced magnetic resonance signals are received by the whole body RF coils 26 or by a local RF coil 28.

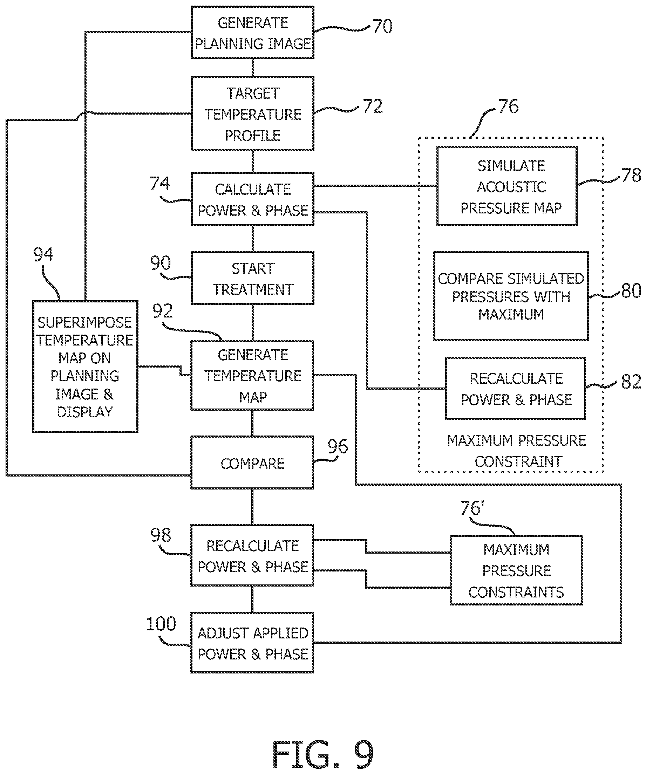

A magnetic resonance scan controller 30 controls the gradient and RF coils to apply a selected magnetic resonance proton density imaging protocol and a thermal imaging protocol such as a fast field echo-echo planar imaging (FFE-EPI) sequence utilizing proton resonance frequency shift (PRFS). A magnetic resonance reconstruction system 32 reconstructs the received resonance signals into a proton-density-weighted planning image 34 prior to treatment and into a series of MR thermal image maps 36 during ultrasonic treatment. A clinician uses a planning terminal 38 to plan the spatial and thermal properties of the mild hyperthermia treatment to provide spatial and thermal treatment profiles, which are stored in a treatment profile memory 40. The ultrasound controller 42 calculates a multi-foci sonication pattern (explained below in greater detail in conjunction with FIG. 9) that achieves the selected spatial and thermal profiles.

The ultrasound probe 14 includes phased array ultrasound transducers 50, such as a 256-element transducer array. The ultrasound probe 14, in the illustrated embodiment, is disposed in a patient support 44, which is movably mounted to transport the patient and the ultrasound transducer into the examination region 22 of the magnetic resonance imaging system 12. Driving electronics including an array of ultrasonic transducer drivers 52 are provided, each driver parallel to a corresponding transducer, for providing the appropriate power and frequency to each ultrasonic transducer individually with an appropriate relative phase to deliver the multi-foci sonication pattern calculated by the ultrasound controller 42. The ultrasound transducers are individually controlled. The driving electronics can be used to direct the multi-foci beam to different spatial locations, different depths, and different relative power levels around the profile. Optionally, a mechanical aiming device 54 is provided for mechanically adjusting the angle and location of the ultrasound probe, hence the relative location of the multi-foci beam within the patient. The driving electronics enable the creation of the desired focal pattern through the generation of simultaneous multiple foci associated with low levels of secondary maxima. In one embodiment, an acoustic coupling 56 is carried by the transducer array to contract and interface with the patient.

The ultrasonic controller 42 and the driving electronics 54 control the array of ultrasound transducers to sonicate any of a multiplicity of freely configurable patterns. For example, the patterns may include a circular array of foci with diameters of the circle from 4-32 mm. To heat a larger volume, two or more of the circles can be sonicated simultaneously or alternately. For more complex patterns, the ultrasound controller 42 calculates the appropriate driving voltages and potential for each of the transducers of the array individually. Alternately, the ultrasound controller can be preprogrammed with a plurality of patterns: one-dimensional, two-dimensional, and/or three-dimensional. These patterns can be scaled and combined, as needed, to match the shape and size of the volume that is to be heated. The operator may specify a target region that is then decomposed into a set of sonication patterns with shapes determined to for the heating to best conform to the target region. The frequency, the sonication target size, and the sonication depth are also adjustable.

The ultrasound controller and the driving electronics control the transducers to generate patterns, which contain a plurality of foci that are sonicated simultaneously. The patterns with a spread, which ranges from about 4 mm to about 32 mm in width are suitable for most clinical applications. However, this width can be increased by adjusting the geometry, or by increasing the number or size of the transducer elements.

By contrast, for thermal ablation, it is desirable to achieve a rapid, highly localized temperature rise in the target region in order to limit undesirable temperature elevation in surrounding tissue. A well-defined heating pattern with a high energy efficiency maximizes the ratio between the ablation volume and near-field heating. Steep temperature gradients improve lesion delineation. Provided that the ablation is complete, temperature uniformity is not necessary.

Consequently, the sweeping of a single high-intensity focused point for increasing the ablation volume is beneficial for ablation. Low-level cavitation and shockwave formation, which are induced at the focus by high acoustic pressure levels may be beneficial in further improving the heating efficiency of the ablation. Collateral damage to surrounding tissues should be avoided.

For mild hyperthermia, the heating strategy is very different. Mechanical effects, such as shock waves, high acoustic pressure gradients, and cavitation, should be at least be minimized if not altogether avoided. Rather, for mild hyperthermia, the goal is merely to elevate the local tissue temperature while avoiding direct tissue damage. In mild hyperthermia, heating uniformity within a relatively narrow temperature range achieves similar bioeffects throughout the target volume. However, due to the low temperatures of mild hyperthermia, thermal damage to the skin or tissues nearby to the target is unlikely.

Multi-foci sonication patterns distribute the acoustic pressure at any given time over a large area. Although this might reduce heating efficiency and cause treatment borders to be less well defined, the distributed acoustic pressure is still advantageous for mild hyperthermia treatments as for example temporal temperature peaks are better avoided. The driving electronics are used to steer and focus the multi-foci beam to the prescribed shape of the target volume.

The ultrasound controller 42 compares the current MR thermal map 36 with the treatment profile 40 and controls the driving electronics to adjust the power delivered to the individual transducers of the ultrasound array to maintain the actual thermal profile as close as possible to the desired treatment profile while keeping peak pressure below a given threshold to avoid mechanical damage. The magnetic resonance system and the magnetic resonance thermal map 36 provide a feedback loop to control the temperature uniformity while limiting the peak pressure in the target region. To minimize the peak pressure, the peak pressure can be estimated using acoustic simulations compatible with a heterogeneous medium, for example, a stochastic ray tracer that is also rapid. In one embodiment, the transducers are driven to create minimal heating in the target region and the feedback loop iteratively brings the actual temperatures up into conformity with the target temperature profile.

The multi-foci mild hyperthermia sonication with MR-HIFU reduces the possible risks associated with high instantaneous pressures while obtaining a homogeneous temperature distribution in the target region. The present technique achieves accurate and precise heating within the target region with significantly lower acoustic pressures and better spatial control over heating compared to a single focus spot sweep sonication technique. The reduction in acoustic pressure and the improvement in spatial control render multi-foci heating as an advantageous tool for mild hyperthermia applications for clinical oncology.

By contrast, binary ablation feedback strategies when applied to mild hyperthermia, e.g., a rapidly scanning spot, are either incapable of maintaining mild hyperthermia for a long duration, incapable of lowering peak acoustic pressures, or result in an inferior accuracy and/or spatiotemporal uniformity of heating.

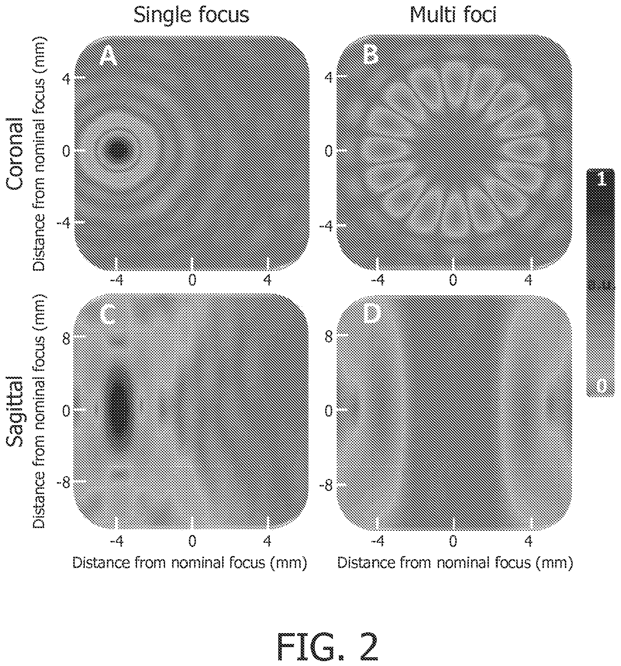

FIG. 2B illustrates an exemplary multi-foci sonication pattern. FIG. 2A, by comparison, illustrates a single focus spot. More specifically, FIGS. 2A &2B are acoustic pressure maps obtained through computer simulations. As can be seen from the pressure scale at the right, the multi-foci sonication pattern of FIG. 2B distributes a smaller pressure over a wide area; whereas, the single spot of FIG. 2A creates a much higher pressure in a more localized area. FIGS. 2C & 2D illustrate the acoustic pressure map in a dimension orthogonal to the acoustic pressure maps of FIGS. 2A & 2B, respectively. In the illustrated embodiment, multi-foci sonication results in about a 70% reduction in peak acoustic pressure.

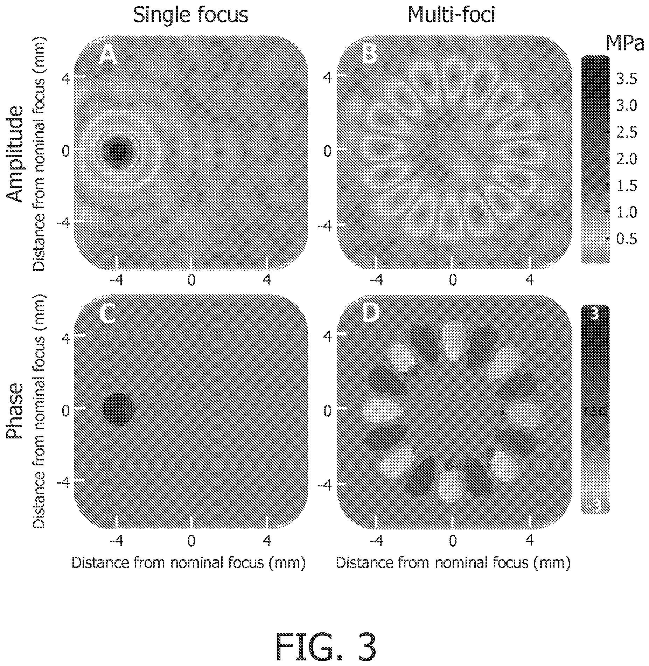

FIGS. 3A & 3B again compare measurements of acoustic pressure maps of a single focal point versus a multi-foci sonication pattern. FIGS. 3C & 3D are phase maps for the single focus and multi-foci sonication patterns.

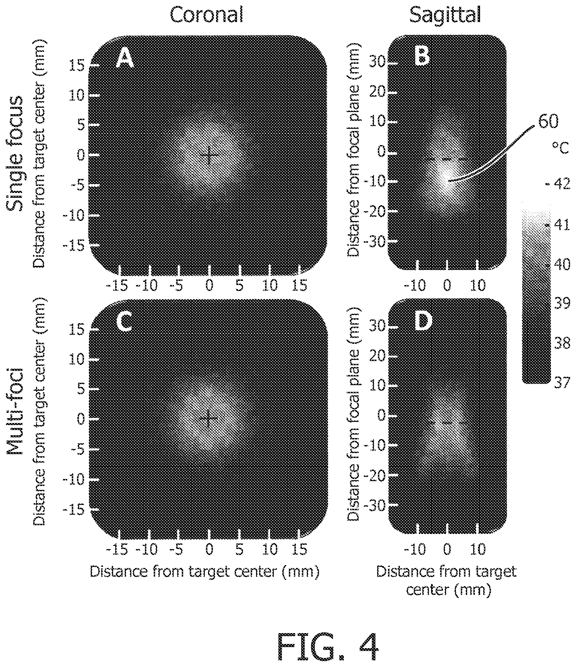

FIGS. 4A-4D compare the heating characteristics of single and multi-foci sonication patterns through computer simulations. The multi-foci and single focus sweep sonications were evaluated in a binary feedback controlled MR-HIFU volumetric mild hyperthermia simulation. As will be seen by comparing FIGS. 4C & 4D for the multi-foci sonication pattern sonication with FIGS. 4A & 4B for the single focus sweep sonication, the multi-foci sonication pattern results in a more homogeneous temperature distribution with less temperature overshoot 60 above the target temperature range, 40.5°-41°C. in the illustrated example.

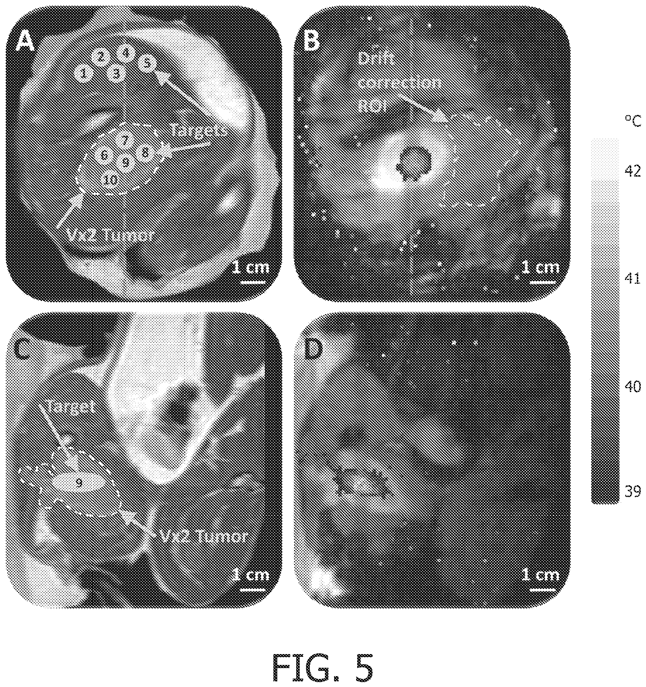

FIGS. 5A-5D illustrate an example of treatment planning and resulting heating (temperature maps) in an *in vivo* study, particularly a RABBIT model with a Vx2 tumor in a thigh muscle using the multi-foci sonication approach. During planning, the tumor is identified with a dashed line on the proton density weighted planning images. Target regions both within the tumor and normal muscle are chosen, note circles 1-5 and 6-10. With reference to FIGS. 5B & 5D, during treatment, temperature maps, preferably in color, are overlaid on the dynamic magnitude images. FIGS. 5B & 5D show a typical temperature distribution after 3 minutes of heating. The ultrasound controller 42 compares these temperature maps with the temperature profile from the treatment profile memory 40 and adjusts the power and phase of the energy applied to the ultrasonic transducer array so as to bring and hold the actual and desired temperature maps in conformity with each other.

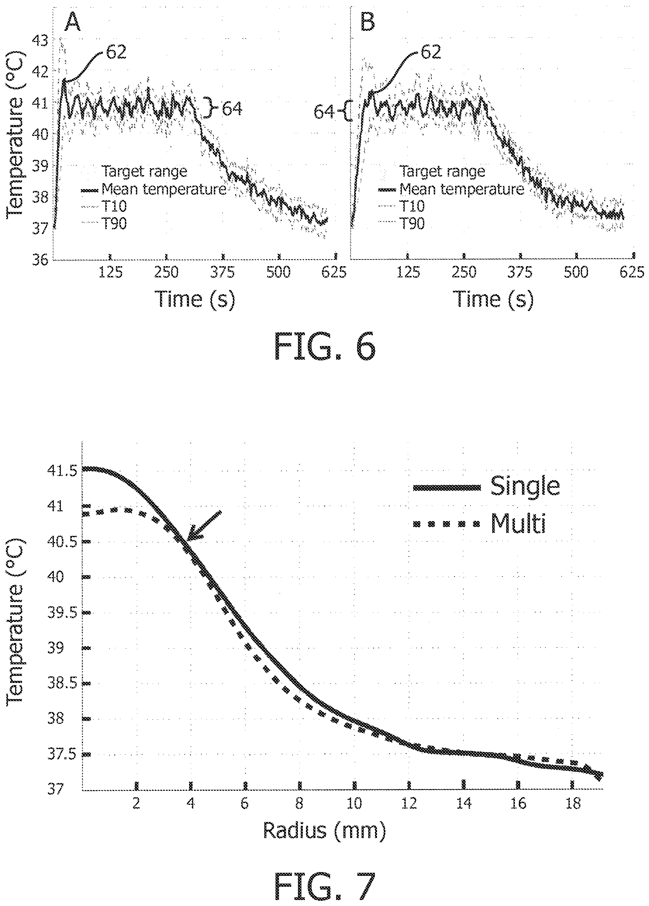

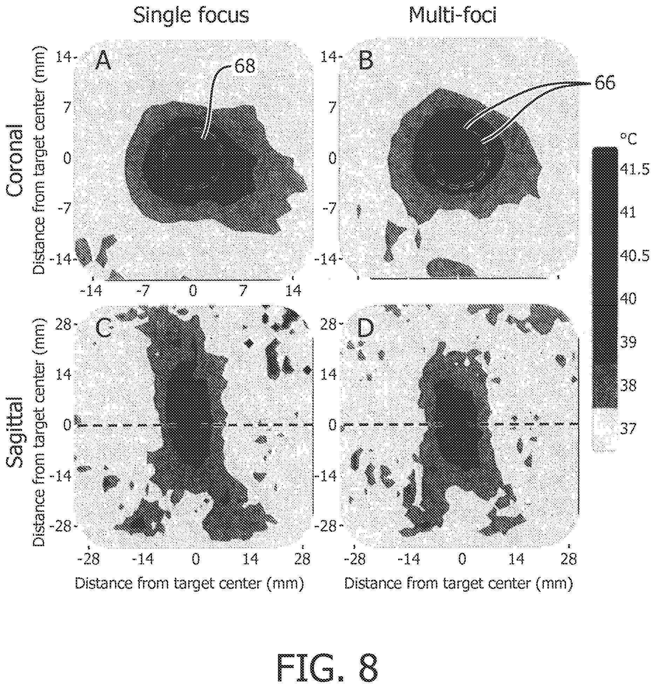

FIGS. 6-8 show mean temperature curves, radial line profiles of temperature in the target region, as well as temperature distribution maps for both multi-foci and single focus sweep methods in *in vivo* studies. Note that the temperature 62 using the multi-foci approach (FIG. 6B) has less overshoot from the target temperature range 64 than a single focus sweep approach illustrated in FIG. 6A. Similarly, a dashed line in FIG. 7 shows that the temperature near the center of the heated region is lower and within the target 40.5-41°C temperature range than the single focus sonication illustrated by the solid line. In FIGS. 8B & 8D, note that the temperature within the target region as denoted by the dashed circle in FIG. 8B is primarily in the 40.5°C. range with only two small regions 66 in the 41°C. range. By contrast, the target region with the single focus sweep sonication results in heating a substantial portion of the target area to 41°C. and brings a significant area 68 near the center of the region to the 41.5°C. range.

With reference again to FIG. 1 and further reference to FIG. 9, the ultrasound controller 42 and the magnetic image reconstruction unit 32 include one or more processors, which are programmed to implement the steps of FIG. 9. The planning image 34 is generated at step 70. The planning image is analyzed by a clinician using the planning console 38 and a treatment profile including a target temperature distribution or profile is generated and stored in the treatment profile memory 40 at step 72. The processors of the ultrasound controller receive the target temperature distribution and, at step 74, calculate the power to be applied to each of the individual transducers of the phased array 50 and the relative phase and frequency or frequencies with which the power is applied in order to generate the multi-foci sonication which will achieve the target temperature distribution.

As discussed above, when using high acoustic pressures, the target temperature may be reached too quickly, e.g., in a matter of microseconds, with respect to the sampling interval (dynamic imaging frame rate), thereby inducing severe overshoots in the temperature. Moreover, the relatively high acoustic pressures may be sufficiently high that the treated tissue could be mechanically damaged by the acoustic pressure waves. At step 76, maximum pressure constraints are applied to the calculation such that the calculated power and relative phase maintain the maximum acoustic pressure below clinician selected constraints. With such constraints, the heating may be slower, e.g., on the order of $10^{ths}$ of a second, but the pressure remains acceptably low. To apply the pressure constraints, the processor can simulate or estimate an acoustic pressure distribution map at step 78. For a phased-array transducer with $N$ elements, the pressure at a given in $M$ control points at position $r = r_m$ with $m = 1, 2, M$ can be written as

$$p(r_m) = \frac{jpck}{2\pi} \sum_{n=1}^{N} u_n \int_{S_n'} \frac{\exp(-jk|r_m - r_n'|)}{|r_m - r_n'|} dS_n'$$

where $j = \sqrt{-1}$, $p$ is the density, c is the speed of sound, $k$ is the wavenumber, $S'$ is the surface of the source, $u$ is the normal velocity of the source surface, and $r$ and $r'$ are the observation and source points, respectively. The maximum from the pressure distribution map is compared with the preselected maximum pressure at step 80. The power and phase to be applied to each ultrasonic transducer is recalculated or adjusted, as necessary, to meet the acoustic pressure constraint at step 82. This process can be iteratively applied.

At step 90, the treatment is started. This may involve injecting chemotherapeutic agents whose activity is enhanced by heat, or drug containing liposomes whose payload is released at a certain temperature threshold. At step 92, the magnetic resonance scan controller 30 controls the magnetic resonance scanner 12 to start generating the temperature maps 36. Each temperature map 36 is optionally superimposed on the planning image at step 94 and displayed to the clinician, e.g., on the planning console 38. At step 96, the generated temperature maps are compared with the target temperature distribution. If the actual and target temperature distributions differ, the power and the relative phase which is applied to the ultrasonic transducer elements is recalculated or adjusted at step 98. The recalculated powers and relative phases are constrained by the maximum pressure constraints at step 76' analogous to step 76 described above. At step 100, the power and relative phase applied to the ultrasonic transducer elements is adjusted. The temperature maps continue to be generated, compared to the target temperature profiles, and the power and relative phase applied to the transducer elements adjusted for the treatment duration which may, for example, be on the order of 30-45 minutes.

The power and phase applied to the transducers of the phased array 50 can be static or can vary with time in order to move the multi-foci sonication pattern. This can even be done midway between the acquisitions of subsequent temperature maps. For example, if the calculated power and phase results in a circular ring of multi-foci points, the ring can be rotated for more even temperature distribution. To cover larger areas, the ring or other patterns of multi-foci are expanded and contracted in radius. The expansion and contraction can be in steps or swept on a continuum.

Calculating the power, phase and frequency to be applied to the individual acoustic transducers is also constrained to maintain pressures in the region between the transducer array and the target region to be kept below preselected maxima. For example, the phasing of the transducers can be selected such that the pressure fields destructively interfere in the region between the transducer array and the target region.

The apparatus described above can advantageously be combined with mechanical movement or angulation of the transducer for heating at multiple individual mechanical transducer positions or angles in order to create a larger heated region. This will be beneficial for heating of larger tumors.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

**Claims**

**1.** A mild hyperthermia treatment apparatus (10) comprising:

an imager (12) which generates a planning image (34);
a phased array of ultrasonic transducers (50);
an array of ultrasonic transducer drivers (52) for individually driving the ultrasonic transducers of the phased array to generate multi-foci sonications in a target region;
one or more processors (30, 32, 34) programmed to:

receive a target temperature profile based on the planning image, and
calculate power, frequency and relative phase for the transducer drivers of the ultrasonic transducer driver array which causes the phased array of ultrasonic transducers to generate a multi-foci sonication pattern configured to heat the target region with the target temperature profile, **characterised in that** the one or more processors is/are further programmed to:
calculate the powers, frequencies, and relative phases and/or a number of foci such that the multi-foci sonication profile maintains acoustic pressures in the target region below maximum acoustic pressures.

**2.** The apparatus according to either one of claim 1, wherein the one or more processors are further programmed to:

simulate an acoustic pressure distribution map based on the calculated powers and relative phases;
compare acoustic pressures of the simulated acoustic pressure distribution map with a preselected maximum acoustic pressure; and
recalculate the powers, and relative phases, frequencies, and/or the number of foci such that acoustic pressures of the simulated acoustic pressure distribution are below the preselected maximum acoustic pressure.

**3.** The apparatus according to any one of claim 1 or 2, wherein the imaging apparatus is a magnetic resonance scanner (12).

**4.** The apparatus according to claim 3, wherein the magnetic resonance scanner (12) includes a scan controller (30) which controls the magnetic resonance scanner to apply a thermometry imaging sequence utilizing proton resonance frequency shift, T1, T2/T2*, diffusion, proton density, or spectroscopic methods.

**5.** The apparatus according to claim 3 or 4, wherein the one or more processors are further programmed to reconstruct received resonance signals from the imaging apparatus into the planning image and a series of temperature maps,

**6.** The appraratus according to claim 5, wherein the one or more processors are further programmed to reconstruct the series of temperature maps in real time during generation of the multi-foci sonications.

**7.** The apparatus according to any one of claims 5-6, wherein the one or more processors are further programmed to after commencement of the mild hyperthermia treatment:

compare a current temperature map from the series of temperature maps with the target temperature profile;
based on temperature differences between the generated temperature map and the target temperature profile, calculating adjustments to the applied powers, frequencies and relative phases; and
adjust the applied powers, frequencies and phases.

**8.** The apparatus according to any one of claims 1-5, wherein the one or more processors are further programmed to:
decompose the target region into a set of multi-foci sonication patterns.

9. The apparatus according to any one of claims 1-8, further including a planning console (38) which displays the planning image and on which a clinician inputs the target temperature profile.

**Patentansprüche**

1. Behandlungsvorrichtung (10) auf der Basis milder Hyperthermie, aufweisend:

einen Bildgeber (12), der eine Planungsabbildung (34) erzeugt;
eine phasengesteuerte Anordnung aus Ultraschallwandlern (50);
eine Anordnung aus Ultraschallwandler-Ansteuereinheiten (52) zum einzelnen Ansteuern der Ultraschallwandler der phasengesteuerten Anordnung zur Erzeugung von multifokalen Beschallungen in einem Zielbereich;
ein oder mehrere Prozessoren (30, 32, 34), der/die programmiert ist bzw. sind, um:

ein Zieltemperaturprofil beruhend auf der Planungsabbildung zu empfangen, und
die Leistung, Frequenz und relative Phase für die Wandler-Ansteuereinheiten der Ultraschallwandler-Ansteueranordnung zu berechnen, was die phasengesteuerte Anordnung aus Ultraschallwandlern ein multifokales Beschallungsmuster erzeugen lässt, das dazu ausgelegt ist, den Zielbereich mit dem Zieltemperaturprofil zu erwärmen,

**dadurch gekennzeichnet, dass** der eine oder die mehreren Prozessoren darüber hinaus programmiert ist bzw. sind, um:
die Leistungen, Frequenzen und relativen Phasen und/oder eine Anzahl von Fokussen zu berechnen, so dass das multifokale Beschallungsprofil Schalldrücke im Zielbereich unterhalb von Maximalschalldrücken hält.

2. Vorrichtung nach Anspruch 1, wobei der eine oder die mehreren Prozessoren darüber hinaus programmiert ist bzw. sind, um:

eine Schalldruck-Verteilungsdarstellung beruhend auf den berechneten Leistungen und relativen Phasen zu simulieren;
Schalldrücke der simulierten Schalldruck-Verteilungsdarstellung mit einem vorausgewählten Maximalschalldruck zu vergleichen; und
die Leistungen, relativen Phasen, Frequenzen und/oder die Anzahl von Fokussen erneut zu berechnen, so dass die Schalldrücke der simulierten Schalldruckverteilung unter dem vorausgewählten Maximalschalldruck liegen.

3. Vorrichtung nach Anspruch 1 oder 2, wobei die Bildgebungsvorrichtung ein Kernspintomograph (12) ist.

4. Vorrichtung nach Anspruch 3, wobei der Kernspintomograph (12) eine Abtaststeuerung (30) aufweist, die den Kernspintomographen steuert, um eine Thermometrie-Bildgebungssequenz anzuwenden, die Protonenresonanz-Frequenzverschiebung, T1, T2/T2*, Diffusion, Protonendichte oder spektroskopische Verfahren nutzt.

5. Vorrichtung nach Anspruch 3 oder 4, wobei der eine oder die mehreren Prozessoren darüber hinaus programmiert ist bzw. sind, um empfangene Resonanzsignale von der Bildgebungsvorrichtung in der Planungsabbildung und eine Reihe von Temperaturdarstellungen zu rekonstruieren.

6. Vorrichtung nach Anspruch 5, wobei der eine oder die mehreren Prozessoren darüber hinaus programmiert ist bzw. sind, um während der Erzeugung der multifokalen Beschallungen die Reihen von Temperaturdarstellungen in Echtzeit zu rekonstruieren.

7. Vorrichtung nach einem der Ansprüche 5-6, wobei der eine oder die mehreren Prozessoren darüber hinaus programmiert ist bzw. sind, um nach Beginn der milden Hyperthermiebehandlung :

eine aktuelle Temperaturdarstellung aus der Reihe von Temperaturdarstellungen mit dem Zieltemperaturprofil zu vergleichen;
beruhend auf Temperaturunterschieden zwischen der erzeugten Temperaturdarstellung und dem Zieltemperaturprofil Änderungen an den angewendeten Leistungen, Frequenzen und relativen Phasen zu berechnen; und
die angewendeten Leistungen, Frequenzen und Phasen einzustellen.

**8.** Vorrichtung nach einem der Ansprüche 1-5, wobei der eine oder die mehreren Prozessoren darüber hinaus programmiert ist bzw. sind, um:
den Zielbereich in eine Gruppe aus multifokalen Beschallungsmustern zu zerlegen.

**9.** Vorrichtung nach einem der Ansprüche 1-8, darüber hinaus eine Planungskonsole (38) aufweisend, die die Planungsabbildung anzeigt und auf der ein Arzt das Zieltemperaturprofil eingibt.

**Revendications**

**1.** Appareil de traitement par hyperthermie douce (10) comprenant :

un système d'imagerie (12) qui génère une image de planification (34) ;
un réseau à commande de phase de transducteurs ultrasoniques (50) ;
un réseau de circuits de commande de transducteur ultrasonique (52) pour commander individuellement les transducteurs ultrasoniques du réseau à commande de phase en vue de générer les ultrasonifications à foyers multiples dans une région cible ;
un ou plusieurs processeurs (30, 32, 34) programmés pour :

recevoir un profil de température cible basé sur l'image de planification ; et
calculer la puissance, la fréquence et la phase relative des circuits de commande de transducteur du réseau de circuits de commande de transducteur ultrasonique amenant le réseau à commande de phase de transducteurs ultrasoniques à générer un modèle d'ultrasonification à foyers multiples configuré pour chauffer la région cible avec le profil de température cible ;
**caractérisé en ce que** le ou les processeurs est/sont en outre programmé(s) pour :
calculer les puissances, les fréquences et les phases relatives et/ou un nombre de points focaux de telle sorte que le profil d'ultrasonification à foyers multiples maintienne les pressions acoustiques dans la région cible en dessous des pressions acoustiques maximales.

**2.** Appareil selon la revendication 1, dans lequel le ou les processeurs sont en outre programmés pour :

simuler une carte de répartition de la pression acoustique basée sur les puissances calculées et les phases relatives ;
comparer les pressions acoustiques de la carte de répartition de la pression acoustique simulée avec une pression acoustique maximale présélectionnée ; et
recalculer les puissances et les phases relatives, les fréquences et/ou le nombre de points focaux de telle sorte que les pressions acoustiques de la répartition de pression acoustique simulée soient en dessous de la pression acoustique maximale présélectionnée.

**3.** Appareil selon l'une quelconque des revendications 1 ou 2, dans lequel l'appareil d'imagerie est un scanner à résonance magnétique (12).

**4.** Appareil selon la revendication 3, dans lequel le scanner à résonance magnétique (12) comprend un dispositif de commande de balayage (30) qui commande le scanner à résonance magnétique pour appliquer une séquence d'images thermométriques utilisant le déplacement de fréquence de résonance protonique T1, T2/T2*, la diffusion, la densité protonique ou les procédés spectroscopiques.

**5.** Appareil selon la revendication 3 ou 4, dans lequel le ou les processeurs sont en outre programmés pour reconstruire les signaux de résonance reçus provenant de l'appareil d'imagerie en une image de planification et en une série de cartes de température.

**6.** Appareil selon la revendication 5, dans lequel le ou les processeurs sont en outre programmés pour reconstruire en temps réel la série de cartes de température pendant la génération des ultrasonifications à foyers multiples.

**7.** Appareil selon l'une quelconque des revendications 5 ou 6, dans lequel le ou les processeurs sont en outre programmés pour, après le commencement du traitement par hyperthermie douce :

comparer une carte de température actuelle provenant des cartes de température avec le profil de température cible ;

sur la base des différences de température entre la carte de température générée et le profil de température cible, calculer les ajustements apportés aux puissances, fréquences et phases relatives appliquées ; et régler les puissances, fréquences et phases appliquées.

8. Appareil selon l'une quelconque des revendications 1 à 5, dans lequel le ou les processeurs sont en outre programmés pour :

décomposer la région cible en modèles d'ultrasonification à foyers multiples.

9. Appareil selon l'une quelconque des revendications 1 à 8, comprenant en outre une console de planification (38) qui affiche l'image de planification et sur laquelle un praticien saisit le profil de température cible.

FIG. 1

FIG. 2

FIG. 3

FIG. 4

FIG. 5

FIG. 6

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120191020 A1

- US 20100030076 A1